# EUROPEAN PATENT APPLICATION

(11) **EP 4 570 903 A1**
(43) Date of publication of application: **18.06.2025**
(21) Application number: 23853003.4
(22) Date of filing: 09.08.2023
(51) Int. Cl.: C12N 5/079

(54) **METHOD FOR PREPARING CEREBELLAR ORGANOID**

(30) Priority: 10.08.2022 KR 20220099741
(71) Applicant: Next & Bio Inc., Seoul 08826 (KR)
(72) Inventor: KWAK, Tae Hwan, Yongin-si Gyeonggi-do 16824 (KR); YANG, Ji Hun, Seoul 04413 (KR)
(74) Representative: Kador & Partner Part mbB
(86) International application number: PCT/KR2023/011791
(87) International publication number: WO 2024/035134

(57) **Abstract**

The present disclosure relates to a method for preparing a cerebellar organoid, comprising: (A) a step of selective differentiation into hindbrain tissue, in which embryoid bodies formed from pluripotent stem cells are cultured in the presence of a hindbrain tissue induction medium including an endogenous WNT secretion inhibitor and a WNT signaling agonist; (B) a step of selective differentiation into dorsal metencephalic tissue, in which the embryoid bodies are cultured in the presence of a dorsal metencephalic tissue induction medium including a sonic hedgehog (SHh) signaling pathway inhibitor; and (C) a basal surface-apex surface polarization step of inducing basal surface-apex surface polarization, in which polarization of the embryoid bodies are induced in the presence of a basal surface-apex surface induction medium including an extracellular matrix-based hydrogel.

## Description

### [Technical Field]

The present disclosure relates to a method for preparing a cerebellar organoid.

### [Background Art]

An organoid, also called 'organ analogue' or 'organ-like body', is an organ-specific cell aggregate prepared by reaggregating and recombining cells isolated from stem cells or organ-origin cells using a 3D culture method. The organoid contains specific cells of the organ for modeling, reproduces specific functions of the organ, and can be spatially organized in a form similar to an actual organ.

In particular, organoids may be prepared to mimic most human organs using pluripotent stem cells that may differentiate into all cells of the human body, and among these, a brain organoid has received a lot of attention as an *in vitro* culture model for mimicking various intractable central nervous system diseases to elucidate the pathogenesis and develop treatments.

For example, Lancaster et al. prepared brain organoids using human induced pluripotent stem cells (Non-Patent Document 1). However, the brain organoids prepared in this way have a problem in that various organs, such as the cerebrum, midbrain, and retina, are mixed in a single organoid, and their sizes are also different.

Most intractable brain diseases are diseases that occur specifically in a specific brain region, and organoids that have the characteristics of a brain region other than a target disease site or have mixed characteristics of various regions cannot accurately mimic brain diseases. Therefore, in order to utilize brain organoids for research on the pathogenesis of intractable brain diseases through modeling, research on developing treatments, etc., it is required to develop a region-specific brain organoid fabrication technology capable of mimicking a specific brain region with high purity.

### [Prior Arts]

### [Non-Patent Document]

(Non-Patent Document 1) Madeline A. Lancaster, Juergen A. Knoblich, Generation of Cerebral Organoids from Human Plurpotent Stem Cells, Nat Protoc. 2014 October; 9(10): 2329-2340.

### [Disclosure]

### [Technical Problem]

An object of the present disclosure is to provide a method for preparing a cerebellar organoid from embryoid bodies derived from pluripotent stem cells.

However, technical objects of the present disclosure are not limited to the aforementioned purpose and other objects which are not mentioned may be clearly understood to those skilled in the art from the following description.

### [Technical Solution]

An aspect of the present disclosure provides a method for preparing a cerebellar organoid, including: (A) a step of selective differentiation into hindbrain tissue, in which embryoid bodies formed from pluripotent stem cells is cultured in the presence of a hindbrain tissue induction medium including an endogenous WNT secretion inhibitor and a WNT signaling agonist;
(B) a step of selective differentiation into dorsal metencephalic tissue, in which the embryoid bodies is cultured in the presence of a dorsal metencephalic tissue induction medium including a sonic hedgehog (SHh) signaling pathway inhibitor; and
(C) a basal surface-apex surface polarization step, in which polarization of the embryoid bodies is induced in the presence of a basal surface-apex surface induction medium including an extracellular matrix-based hydrogel.

### [Advantageous Effects]

According to the embodiment of the present disclosure, in the case of using the method for preparing the cerebellar organoid, it is possible to prepare a cerebellar organoid that mimics a cerebellar region with high purity and to contribute to the development of research on the pathogenesis of cerebellar-related diseases, research on the development of treatment methods, and the like using the same.

### [Description of Drawings]

FIG. 1 shows results of expression of forebrain region-specific genes according to a concentration of a WNT signaling agonist in a hindbrain tissue induction medium of Example.
FIG. 2 shows results of expression of hindbrain region-specific genes according to a concentration of a WNT signaling agonist in a hindbrain tissue induction medium of Example.
FIG. 3 shows results of expression of spinal cord region-specific genes according to a concentration of a WNT signaling agonist in a hindbrain tissue induction medium of Example.
FIG. 4 shows results of expression of dorsal hindbrain region-specific genes according to a concentration of an SHh signaling pathway inhibitor in a dorsal metencephalic tissue induction medium of Example.
FIG. 5 shows a developmental state of an embryoid bodies on day 7 after selective differentiation into dorsal metencephalic tissue according to a concentration of the SHh signaling pathway inhibitor in the dorsal metencephalic tissue induction medium of Example.
FIG. 6 shows a process of preparing a cerebellar organoid according to Example in a time series from embryoid bodies formation (Day 0).
FIG. 7 shows a process of developing an embryoid bodies in the process of preparing the cerebellar organoid according to Example observed through a phase microscope.
FIG. 8 shows results of observing a cerebellar organoid on Day 35 from the date of embryoid bodies formation according to the method for preparing the cerebellar organoid according to Example, using immunofluorescence staining.

### [Best Mode]

Hereinafter, the present disclosure will be described in detail.

The present disclosure may have various modifications and various exemplary embodiments, and specific exemplary embodiments will be illustrated in the drawings and described in detail in the detailed description.

However, the present disclosure is not limited to specific exemplary embodiments, and it should be understood that the present disclosure covers all the modifications, equivalents and replacements included within the idea and technical scope of the present disclosure. In describing the present disclosure, when it is determined that a detailed description of related known arts may obscure the gist of the present disclosure, the detailed description will be omitted.

Terms used herein are used only to describe specific exemplary embodiments, and are not intended to limit the present disclosure. A singular expression includes a plural expression unless otherwise defined differently in a context.

In the present disclosure, it should be understood that term "including" or "having" indicates that a feature, a number, a step, an operation, a component, a part or the combination thereof described in the specification is present, but does not exclude a possibility of presence or addition of one or more other features, numbers, steps, operations, components, parts or combinations, in advance.

An embodiment of the present disclosure provides a method for preparing a cerebellar organoid, including: (A) a step of selective differentiation into hindbrain tissue, in which embryoid bodies formed from pluripotent stem cells are cultured in the presence of a hindbrain tissue induction medium including an endogenous WNT secretion inhibitor and a WNT signaling agonist;
(B) a step of selective differentiation into dorsal metencephalic tissue, in which the embryoid bodies are cultured in the presence of a dorsal metencephalic tissue induction medium including a sonic hedgehog (SHh) signaling pathway inhibitor; and
(C) a basal surface-apex surface polarization step, in which polarization of the embryoid bodies are induced in the presence of a basal surface-apex surface induction medium including an extracellular matrix-based hydrogel.

According to one embodiment of the present disclosure, through the step (A) of selective differentiation into the hindbrain tissue, the embryoid bodies formed from the pluripotent stem cells may differentiate into a cell aggregate that mimics hindbrain characteristics with high purity. Specifically, the cell aggregate mimicking the hindbrain characteristics may be an organoid mimicking the hindbrain characteristics with high purity. The cell aggregate or organoid mimicking the hindbrain characteristics with high purity may mean a state in which the mixing of tissues exhibiting characteristics of other brain regions such as the cerebrum, striatum, and midbrain is minimized.

According to one embodiment of the present disclosure, the step (A) of selective differentiation into the hindbrain tissue may uniformly induce patterning into hindbrain tissue throughout the embryoid bodies, while inducing differentiation of the embryoid bodies into the neuroectoderm.

According to one embodiment of the present disclosure, the endogenous WNT secretion inhibitor may be a material that inhibits the secretion of a WNT signaling material naturally expressed in the embryoid bodies. The endogenous WNT secretion inhibitor may control the heterogeneous expression of an uncontrollable WNT signaling pathway by inhibiting the activation of the WNT signaling pathway due to a WNT protein spontaneously expressed within the embryoid bodies. Therefore, the embryoid bodies may be selectively and homogeneously differentiated into the hindbrain by combining and applying the endogenous WNT secretion inhibitor and the WNT signaling agonist to be described below. The endogenous WNT secretion inhibitor may include a material capable of performing a mechanism as a porcupine O-acyltransferase (PORCN) inhibitor. Specifically, the endogenous WNT secretion inhibitor may include at least one selected from the group consisting of IWP-2, LGK-974, ETC-159, GNF6231, WNT-C59, and WNT974. More specifically, the endogenous WNT secretion inhibitor may include IWP-2. More specifically, the endogenous WNT secretion inhibitor may effectively neutralize the activity of the WNT signaling pathway by inhibiting the secretion of naturally expressed WNT signaling materials, and simultaneously may not affect the activity thereof even if the WNT signaling agonist is treated. That is, the endogenous WNT secretion inhibitor may effectively control the heterogeneous formation of the WNT signaling pathway by inhibiting a process of transforming the endogenous WNT protein spontaneously expressed in the embryoid bodies into a form secreted out of a cell from the endoplasmic reticulum. In contrast, materials such as IWR-1-endo may have a problem of reducing an effect of the WNT signaling agonist to be described below, by inhibiting signaling in a lower cascade of the WNT signaling pathway.

According to one embodiment of the present disclosure, the hindbrain tissue induction medium may include an endogenous WNT secretion inhibitor in a concentration range that exhibits an effect equivalent to the effect exhibited by IWP-2 in a concentration range of 0.1 µM or more and 2 µM or less. Specifically, the hindbrain tissue induction medium may include an endogenous WNT secretion inhibitor in a concentration range that exhibits an effect equivalent to the effect exhibited by IWP-2 in a concentration range of 0.5 µM or more and 2 µM or less, 0.5 µM or more and 1.5 µM or less, or 0.8 µM or more and 1.2 µM or less. In addition, according to one embodiment of the present disclosure, the hindbrain tissue induction medium may include IWP-2 in a concentration range of the endogenous WNT secretion inhibitor. When the concentration of the endogenous WNT secretion inhibitor is within the above range, the formation of the WNT signaling pathway spontaneously expressed in the embryoid bodies may be effectively inhibited. Specifically, when the concentration of the endogenous WNT secretion inhibitor is less than the above range, an effect of controlling the heterogeneous expression of the uncontrollable WNT signaling pathway may be minimal, resulting in the formation of a heterogeneous WNT signaling pathway. In addition, when the concentration of the endogenous WNT secretion inhibitor is more than the above range, excessive inhibition of the WNT signaling pathway may result in decreased cell division and/or decreased cell viability.

According to one embodiment of the present disclosure, the WNT signaling agonist may uniformly impart hindbrain region specificity to the entire embryoid bodies. That is, as described above, while the endogenous WNT secretion inhibitor inhibits the generation of the uncontrollable WNT signaling material in the embryoid bodies, the WNT signaling agonist may uniformly control a WNT signal intensity in the entire embryoid bodies to have uniform hindbrain specificity in the entire embryoid bodies.

According to one embodiment of the present disclosure, the WNT signaling agonist may include at least one of a protein-based WNT signaling agonist and a compound-based WNT signaling agonist. Specifically, the protein-based WNT signaling agonist may include at least one selected from the group consisting of Wnt family member 1 (WNT-1), Wnt family member 3A (WNT-3a), and R-spondin-1 (RSPO1). In addition, the compound-based WNT signaling agonist may include a material that performs the mechanism of a GSK-3 beta inhibitor, and specifically, may include at least one selected from the group consisting of CHIR99021, CP21R7, CHIR98014, LY2090314, kenpaullone, AR-AO144-18, TDZD-8, SB216763, BIO, TWS-119, and SB415286. Specifically, the WNT signaling agonist may be CHIR99021.

According to one embodiment of the present disclosure, the selective differentiation into the hindbrain region may be induced by optimizing the concentration of the WNT signaling agonist in the hindbrain tissue induction medium. Specifically, the concentration of the WNT signaling agonist for inducing selective differentiation of the embryoid bodies into the hindbrain region may be as follows.

According to one embodiment of the present disclosure, the hindbrain tissue induction medium may include a WNT signaling agonist in a concentration range that exhibits an effect equivalent to the effect exhibited by CHIR99021 in a concentration range of more than 1 µM and less than 5 µM. Specifically, the hindbrain tissue induction medium may include a WNT signaling agonist in a concentration range that exhibits an effect equivalent to the effect exhibited by CHIR99021 in a concentration range of 1.5 µM or more and 4.5 µM or less, 1.5 µM or more and 4 µM or less, 1.5 µM or more and 3 µM or less, or 1.5 µM or more and 2.5 µM or less. More specifically, the hindbrain tissue induction medium may include the WNT signaling agonist at a concentration that specifically expresses at least one gene of LMX1A and LMX1B. In addition, according to one embodiment of the present disclosure, the hindbrain tissue induction medium may include CHIR99021 in a concentration range of the WNT signaling agonist. When the concentration of the WNT signaling agonist is within the above range, the expression of hindbrain region-specific genes (e.g., LMX1A and/or LMX1B genes) is effectively greatly increased, so that the embryoid bodies may be uniformly differentiated into a cell population having hindbrain specificity. When the concentration of the WNT signaling agonist is less than the above range, the expression of genes (e.g., FOXG1, LHX2, and/or DLX2 genes) specific to the forebrain region rather than the hindbrain region may be increased. In addition, when the concentration of the WNT signaling agonist is more than the above range, the expression of genes (e.g., HOXB4, HOXC9 and/or HOXA1 genes) specific to the spinal cord region rather than the hindbrain region may be increased.

According to one embodiment of the present disclosure, in the step (A) of selective differentiation into the hindbrain tissue, at least one gene of LMX1A and LMX1B may be expressed.

According to one embodiment of the present disclosure, the pluripotent stem cells may be human-derived embryonic stem cells or human-derived induced pluripotent stem (iPS) cells. The pluripotent stem cells refer to stem cells that can differentiate into three germ layers of endoderm, mesoderm, and ectoderm (pluripotency), and may include induced pluripotent stem cells (iPS) and adult stem cells, which have the capabilities, as well as the embryonic stem cells.

According to one embodiment of the present disclosure, the step (A) of selective differentiation into the hindbrain tissue may include: a1) a step of forming embryoid bodies from pluripotent stem cells in an embryoid body formation medium; and a2) a step of adding a hindbrain tissue induction medium containing an endogenous WNT secretion inhibitor and a WNT signaling agonist to the embryoid body formation medium.

According to one embodiment of the present disclosure, the embryoid body formation medium may be a liquid medium, and may be applied as long as 3D culture of the pluripotent stem cells is enabled. Furthermore, the embryoid body formation medium may include various known additives for culturing the pluripotent stem cells, depending on the purpose.

According to one embodiment of the present disclosure, the hindbrain tissue induction medium may include a known culture solution and additives capable of culturing the embryoid bodies, and may further include the endogenous WNT secretion inhibitor and the WNT signaling agonist.

According to one embodiment of the present disclosure, the added amount of the hindbrain tissue induction medium may be 0.1 to 2 times greater than a volume of the embryoid body formation medium. More specifically, the added amount of the hindbrain tissue induction medium may be equivalent to the volume of the embryoid body formation medium.

According to one embodiment of the present disclosure, the step (A) of selective differentiation into the hindbrain tissue may further include a3) a step of removing a portion of the entire medium and adding the hindbrain tissue induction medium at one time point of 1 to 3 days. Specifically, the step a3) may be removing the medium equivalent to the amount of hindbrain tissue induction medium added in the step a2) approximately every two days, and adding an equivalent amount of hindbrain tissue induction medium. Through this, nutrients may be supplied to the embryoid bodies, and the endogenous WNT secretion inhibitor and the WNT signaling agonist may effectively act.

According to one embodiment of the present disclosure, the step (A) of selective differentiation into the hindbrain tissue may be performed for a period of 4 to 10 days. Specifically, the step (A) of selective differentiation into the hindbrain tissue may be performed for a period of 5 to 8 days, a period of 5 to 7 days, or a period of about 6 days.

According to one embodiment of the present disclosure, through the step (B) of selective differentiation into the dorsal metencephalic tissue, the embryoid bodies derived from the pluripotent stem cells may differentiate into a cell aggregate that mimics dorsal hindbrain characteristics with high purity. Specifically, the cell aggregate mimicking the dorsal hindbrain characteristics may be an organoid mimicking the dorsal hindbrain characteristics with high purity. The cell aggregate or organoid mimicking the dorsal hindbrain characteristics with high purity may mean a state in which the mixing of tissues exhibiting characteristics of other brain regions such as the cerebrum, striatum, and midbrain is minimized.

According to one embodiment of the present disclosure, the step (B) of selective differentiation into the dorsal metencephalic tissue may uniformly induce patterning into dorsal metencephalic tissue throughout the embryoid bodies, while inducing differentiation of the embryoid bodies into the neuroectoderm.

According to one embodiment of the present disclosure, the embryoid bodies derived from the pluripotent stem cells may be embryoid bodies differentiating into the hindbrain tissue. Specifically, the step (B) of selective differentiation into the dorsal metencephalic tissue may induce the embryoid bodies differentiating into the hindbrain tissue such as the pons and cerebellum to be specified as dorsal metencephalic tissue during the differentiation process to have higher cerebellar specificity.

According to one embodiment of the present disclosure, the step (B) of selective differentiation into the dorsal metencephalic tissue may include a step of adding the dorsal metencephalic tissue induction medium to a medium in which the embryoid bodies derived from the pluripotent stem cells are being cultured. Specifically, the medium in which the embryoid bodies derived from the pluripotent stem cells are being cultured may include a hindbrain tissue induction medium. Specifically, the step (B) of selective differentiation into the dorsal metencephalic tissue may be performed in an overlapping manner with the treatment of the embryoid bodies differentiating into specific tissues. More specifically, the step (B) of selective differentiation into the dorsal metencephalic tissue may be performed in an overlapping manner with the step of selective differentiation into the hindbrain tissue using the hindbrain tissue induction medium. At this time, the hindbrain tissue induction medium may include an endogenous WNT secretion inhibitor and a WNT signaling agonist.

According to one embodiment of the present disclosure, the SHh signaling pathway inhibitor may be a material that serves to inhibit the SHh signaling pathway that is naturally activated in some cells within the embryoid bodies. The SHh signaling pathway inhibitor may inhibit the activation of the heterogeneous and uncontrollable SHh signaling pathway by inhibiting the activation of the SHh signaling pathway caused by a protein that generates an SHh signal spontaneously expressed within the embryoid bodies. That is, the embryoid bodies may be selectively and homogeneously differentiated into dorsal metencephalic tissue by inhibiting the endogenous SHh signal by the SHh signaling pathway inhibitor.

According to one embodiment of the present disclosure, the SHh signaling pathway inhibitor may include at least one selected from the group consisting of cyclopamine, itraconazole, robotnikinin, cerulenin, and GANT61. Specifically, the SHh signaling pathway inhibitor may be cyclopamine.

According to one embodiment of the present disclosure, the dorsal metencephalic tissue induction medium may include an SHh signaling pathway inhibitor in a concentration range that exhibits an effect equivalent to the effect exhibited by cyclopamine in a concentration range of more than 10 µM and 30 µM or less. Specifically, the dorsal metencephalic tissue induction medium may include an SHh signaling pathway inhibitor in a concentration range that exhibits an effect equivalent to the effect exhibited by cyclopamine in a concentration range of more than 10 µM and less than 30 µM, 12 µM or more and less than 30 µM, 12 µM or more and 30 µM or less, 15 µM or more and 25 µM or less, or 20 µM or more and 25 µM or less. More specifically, the dorsal metencephalic tissue induction medium may include the SHh signaling pathway inhibitor at a concentration that specifically expresses at least one gene of PAX7, OLIG3, ATOH1, BARHL1, PTF1A and SKOR2. In addition, the dorsal metencephalic tissue induction medium may include cyclopamine in a concentration range of the SHh signaling pathway inhibitor. When the concentration of the SHh signaling pathway inhibitor is within the above range, the expression of dorsal hindbrain region-specific genes (e.g., PAX7, OLIG3, ATOH1, BARHL1, PTF1A and/or SKOR2) is effectively greatly increased, so that the embryoid bodies may be uniformly differentiated into a cell population having dorsal hindbrain specificity. When the concentration of the SHh signaling pathway inhibitor is less than the above range, the expression of the dorsal hindbrain-specific gene as described above may be minimal. In addition, when the concentration of the SHh signaling pathway inhibitor is more than the above range, the excessive concentration of the SHh signaling pathway inhibitor may inhibit cell division within the embryoid bodies, causing a problem in which the embryoid bodies do not develop as much as necessary and dies.

According to one embodiment of the present disclosure, in the step (B) of selective differentiation into the dorsal metencephalic tissue, at least one gene of PAX7, OLIG3, ATOH1, BARHL1, PTF1A, and SKOR2 may be expressed.

According to one embodiment of the present disclosure, the step of selective differentiation into the dorsal metencephalic tissue may further include b1) a step of adding the dorsal metencephalic tissue induction medium to a medium in which the embryoid bodies are being cultured.

According to one embodiment of the present disclosure, the step of selective differentiation into the dorsal metencephalic tissue may be performed in an overlapping manner with the step of selective differentiation into the hindbrain tissue. Specifically, during the period in which the step of selective differentiation into the dorsal metencephalic tissue is performed in an overlapping manner with the step of selective differentiation into the hindbrain tissue, the dorsal metencephalic tissue induction medium may further include the SHh signaling pathway inhibitor described above, in the hindbrain tissue induction medium.

According to one embodiment of the present disclosure, the added amount of the dorsal metencephalic tissue induction medium may be 0.1 to 2 times greater than a volume of the culturing medium. More specifically, the added amount of the dorsal metencephalic tissue induction medium may be equivalent to the volume of the culturing medium.

According to one embodiment of the present disclosure, the step of selective differentiation into the dorsal metencephalic tissue may further include b2) a step of removing a portion of the entire medium and adding the dorsal metencephalic tissue induction medium at one time point of 1 to 3 days. Specifically, the step b2) may be removing the medium equivalent to the amount of dorsal metencephalic tissue induction medium added in step b1) approximately every two days, and adding an equivalent amount of dorsal metencephalic tissue induction medium. Through this, the nutrients may be supplied to the embryoid bodies, and the SHh signaling pathway inhibitor may effectively act. In addition, when overlapping with the step of selective differentiation into the hindbrain tissue, the SHh signaling pathway inhibitor may effectively act together with the endogenous WNT secretion inhibitor and the WNT signaling agonist.

According to one embodiment of the present disclosure, the step (B) of selective differentiation into the dorsal metencephalic tissue may be performed for a period of 4 to 10 days. Specifically, the step (B) of selective differentiation into the dorsal metencephalic tissue may be performed for a period of 5 to 8 days, a period of 5 to 7 days, or a period of about 6 days.

According to one embodiment of the present disclosure, the step (B) of selective differentiation into the dorsal metencephalic tissue may be performed for a period of 4 to 10 days from 3 to 5 days after the step (A) of selective differentiation into the hindbrain tissue is performed. For example, when the time point of extracting and culturing the pluripotent stem cells is counted as day 0, the embryoid bodies (day 1) is formed by culturing the cells for about 1 day, and then the step of selective differentiation into the hindbrain tissue may be performed from day 1 to about day 7 to about day 9, and the step of selective differentiation into the dorsal metencephalic tissue may be performed from about day 3 to about day 12.

According to one embodiment of the present disclosure, the basal surface-apex surface polarization step (C) induces the polarity of the surface of the embryoid bodies during culturing to be basal, and may polarize the outer surface region and the inner surface region of the embryoid bodies to the basal surface and the apex surface, respectively. Through this, the embryoid bodies during culturing may be specialized into cerebellar-specific tissue by further developing neuroepithelial tissue. To perform this role, the basal surface-apex surface induction medium may include a material capable of imparting the polarity of neuroepithelial tissue to the embryoid bodies during culturing. For example, the basal surface-apex surface induction medium may perform the role by including an extracellular matrix-based hydrogel in a concentration range of 1 vol% or more and 10 vol% or less.

According to one embodiment of the present disclosure, the extracellular matrix-based hydrogel may include extracellular matrix-based materials such as laminin and collagen. For example, the extracellular matrix-based hydrogel may be Matrigel.

According to one embodiment of the present disclosure, the basal surface-apex surface polarization step (C) may be performed for a period of 1 to 5 days. Specifically, the basal surface-apex surface polarization step (C) may be performed for a period of 1 to 4 days, 2 to 4 days, or about 3 days.

According to one embodiment of the present disclosure, the step (A) of selective differentiation into the hindbrain tissue may be performed for a period of 4 to 10 days, the step (B) of selective differentiation into the dorsal metencephalic tissue may be performed for a period of 4 to 10 days, and the basal surface-apex surface polarization step (C) may be performed for a period of 1 to 5 days.

According to one embodiment of the present disclosure, the step (A) of selective differentiation into the hindbrain tissue, the step (B) of selective differentiation into the dorsal metencephalic tissue, and the basal surface-apex surface polarization step (C) may be each performed in an overlapping manner to effectively complete patterning into the cerebellum at each step as a process to be performed through each independent element control. The step (A) of selective differentiation into the hindbrain tissue and the step (B) of selective differentiation into the dorsal metencephalic tissue may be performed in an overlapping manner for a period of 1 to 5 days. Specifically, the step (A) of selective differentiation into the hindbrain tissue and the step (B) of selective differentiation into the dorsal metencephalic tissue may be performed in an overlapping manner for a period of 1 to 4 days, 2 to 4 days, or about 3 days.

According to one embodiment of the present disclosure, the step (B) of selective differentiation into the dorsal metencephalic tissue and the basal surface-apex surface polarization step (C) may be performed in an overlapping manner for a period of 1 to 5 days. Specifically, the step (B) of selective differentiation into the dorsal metencephalic tissue and the basal surface-apex surface polarization step (C) may be performed in an overlapping manner for a period of 1 to 4 days, 2 to 4 days, or about 3 days.

According to one embodiment of the present disclosure, the method for preparing the cerebellar organoid may further include (D) a cerebellar organoid maturation step of maturing the embryoid bodies into a cerebellar organoid in the presence of a cerebellar maturation medium including at least one additive selected from the group consisting of a brain-derived neurotrophic factor (BDNF), a glial cell line-derived neurotrophic factor (GDNF), cAMP, and ascorbic acid.

The cerebellar organoid maturation step (D) may be a step for completing the characteristics of the cerebellar organoid through stepwise signaling material treatment. Specifically, the cerebellar organoid maturation step (D) may be a process of treating for differentiating the cells within the embryoid bodies through steps (A) to (C) into neuroepithelial tissue suitable for cerebellar tissue so as to be cultured as cerebellar-compatible tissue. To obtain mature cerebellar organoids, the cerebellar organoid maturation step (D) may be performed for a period of 10 days, 20 days, or 30 days or more.

According to one embodiment of the present disclosure, the ratio of cerebellar-compatible tissue in the entire prepared cerebellar organoid tissue may be at least 90%. Specifically, the ratio of cerebellar-compatible tissue in the entire prepared cerebellar organoid tissue may be at least 92%, 94%, or 95%. According to one embodiment of the present disclosure, the cerebellar organoid may be an organoid in which the characteristics of cerebellar tissue are implemented with high purity, and may have minimized mixing of tissue from other brain regions such as the cerebrum, striatum, and midbrain. Through this, the development of cerebellar-specific neural cells and neuroglia cells may be observed through cerebellar organoids obtained by the method for preparing the cerebellar organoid according to one embodiment of the present disclosure. In addition, the cerebellar organoid according to the present disclosure, in which the characteristics of cerebellar tissue are implemented with high purity, can be utilized in various applications, such as in research on the cerebellar development process and research on mimicking cerebellar-specific diseases.

The present disclosure may have various modifications and various embodiments, and specific embodiments will be hereinafter illustrated in the drawings and described in detail in the detailed description. However, the present disclosure is not limited to specific embodiments, and it should be understood that the present disclosure covers all the modifications, equivalents and replacements within the idea and technical scope of the present disclosure. In describing the present disclosure, when it is determined that a detailed description of related known arts may obscure the gist of the present disclosure, the detailed description will be omitted.

### [Examples]

### [Formation of embryoid bodies] (embryoid bodies on day 0) (Day 0)

Pluripotent stem cells were cultured in an embryoid body formation medium as shown in Table 1 below.

**[Table 1]**

| Ingredient | Product name | Concentration | Volume |
|---|---|---|---|
| DMEM/F12 | Corning, 10-090-CV | 1X | 37 ml |
| KSR | ThermoFisher, 10828-028 | 1X | 10 ml |
| Penicillin-Streptomycin | ThermoFisher, 15140-122 | 100X | 0.5 ml |
| Glutamax | ThermoFisher, 35050-061 | 100X | 0.5 ml |
| NEAA | ThermoFisher, 11140-050 | 100X | 0.5 ml |
| FBS | ThermoFisher, 16000-044 | 1X | 1.5 ml |
| Heparin | Sigma-Aldrich, H3149 | 1 mg/ml | 50 ul |
| Betamercaptoethanol | ThermoFisher, 21985-023 | 1,000X | 50 ul |
| bFGF | Peprotech, 100-18B | 4 µg/ml | 50 ul |
| Y 27632 | Peprotech, 1293823 | 50 mM | 50 ul |
| Total | 50 ml | | |

When the density of pluripotent stem cells reached approximately 80%, residues on the cell surface were washed using D-PBS and separated into each cell. Furthermore, the cells were diluted to contain approximately 100,000 cells per 10 ml of an embryoid body formation medium, and approximately 100 µl of the medium containing the cells was injected into each well of an ultra-low-attachment U bottom 96 well plate (Corning, 7007). Then, the cells were added in an incubator under a CO₂ atmosphere at 37°C and cultured for about 24 hours to form embryoid bodies.

[Selective differentiation into hindbrain tissue] (embryoid bodies on days 1 to 4)

### (Day 1~4)

After the embryoid bodies were formed as described above, after about one day, it was confirmed whether one spherical embryoid bodies with a clean surface was formed well in each well. A hindbrain tissue induction medium containing IWP-2 as an endogenous WNT secretion inhibitor at a concentration of 1 µM and CHIR99021 as a WNT signaling agonist at concentrations of 1 µM, 2 µM, 3 µM, and 5 µM, respectively was prepared, and then about 100 µl of the hindbrain tissue induction medium was added to each well containing the embryoid bodies.

About 100 µl of the existing medium was removed about once every two days, about 100 µl of the hindbrain tissue induction medium was added, and the embryoid bodies were cultured for 7 days to confirm whether selective differentiation of the embryoid bodies into hindbrain tissue was performed well.

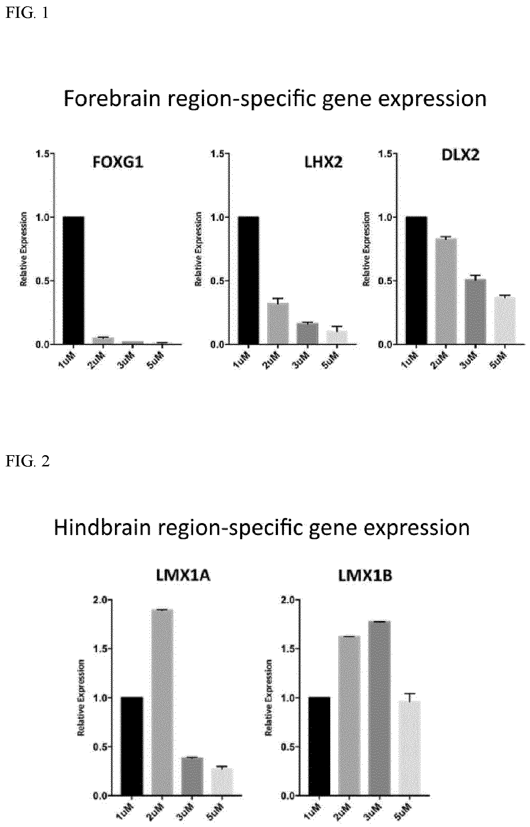

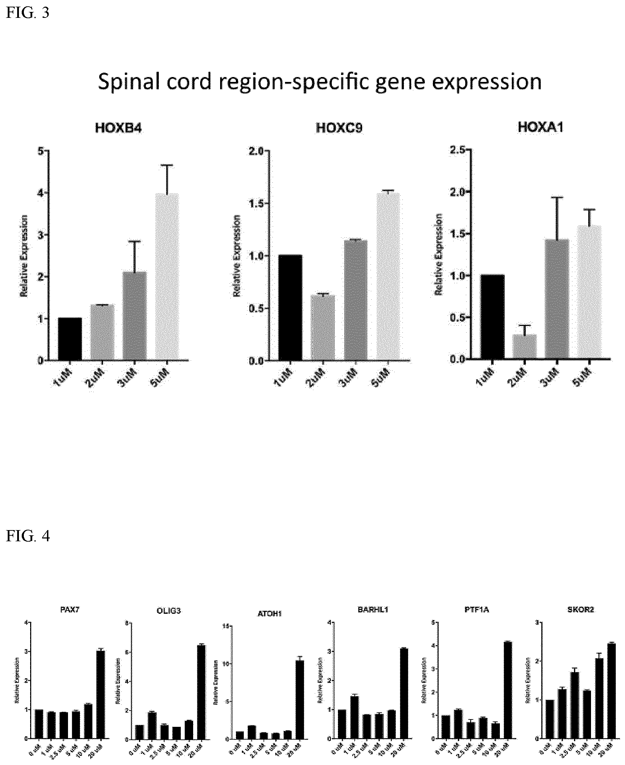

FIG. 1 shows results of expression of forebrain region-specific genes according to a concentration of a WNT signaling agonist in a hindbrain tissue induction medium of Example. FIG. 2 shows results of expression of hindbrain region-specific genes according to a concentration of a WNT signaling agonist in a hindbrain tissue induction medium of Example. Further, FIG. 3 shows results of expression of spinal cord region-specific genes according to a concentration of a WNT signaling agonist in a hindbrain tissue induction medium of Example.

According to the results of FIGS. 1 to 3, it was confirmed that the expression of hindbrain region-specific genes was strongly induced in the hindbrain tissue induction medium containing CHIR99021 in a concentration range of more than 1 µM and less than 5 µM. Furthermore, it was confirmed that the expression intensity of hindbrain region-specific genes was much stronger than those of other brain region-specific genes in the hindbrain tissue induction medium containing CHIR99021 in a concentration range of 2 µM to 3 µM. In contrast, in the case of the hindbrain tissue induction medium containing CHIR99021 at a level of approximately 1 µM, it was confirmed that the expression of forebrain region-specific genes was stronger than that of other brain regions, and in the case of the hindbrain tissue induction medium containing CHIR99021 at a level of approximately 5 µM, it was confirmed that the expression of spinal cord region-specific genes was stronger than that of other brain regions.

Accordingly, the selective differentiation of the embryoid bodies into hindbrain tissue was performed as described above using the hindbrain tissue induction medium containing IWP-2 as the endogenous WNT secretion inhibitor at a concentration of 1 µM and CHIR99021 as the WNT signaling agonist at concentrations of 2 µM to 3 µM, and the next step was prepared.

### [Selective differentiation into dorsal metencephalic tissue] (embryoid bodies on days 4 to 7) (Day 4-7)

A dorsal metencephalic tissue induction medium was prepared by adding cyclopamine as an SHh signaling pathway inhibitor to the hindbrain tissue induction medium in the concentration range of 0 µM, 15 µM, 20 µM, 25 µM, 30 µM, 35 µM, 40 µM, 45 µM, and 50 µM. Then, for about 3 days from day 4 after performing the selective differentiation into the hindbrain tissue, about 100 µl of the existing medium was removed about once every 2 days, and about 100 µl of the dorsal metencephalic tissue induction medium prepared as above was added, thereby simultaneously inducing selective differentiation of the embryoid bodies into hindbrain tissue and selective differentiation into dorsal metencephalic tissue.

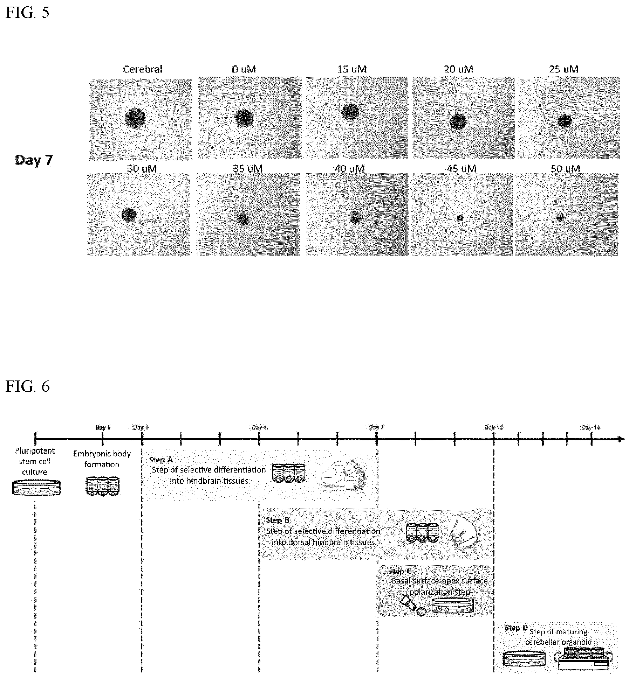

FIG. 4 shows results of expression of dorsal hindbrain region-specific genes according to a concentration of an SHh signaling pathway inhibitor in a dorsal metencephalic tissue induction medium of Example. In addition, FIG. 5 shows a developmental state of embryoid bodies on day 7 after selective differentiation into dorsal metencephalic tissue according to a concentration of the SHh signaling pathway inhibitor in the dorsal metencephalic tissue induction medium of Example.

According to the results in FIG. 4, when the concentration of cyclopamine as an SHh signaling pathway inhibitor was 20 µM or higher, it was confirmed that the expression levels of genes PAX7, OLIG3, ATOH1, BARHL1, PTF1A, and SKOR2, capable of confirming the differentiation into dorsal metencephalic tissue, were increased rapidly. In addition, according to the results of FIG. 5, it was confirmed that when the concentration of cyclopamine as the SHh signaling pathway inhibitor was more than 30 µM, the embryoid bodies no longer grew and died. Through this, it was found that when the concentration of the SHh signaling pathway inhibitor in the dorsal metencephalic tissue induction medium was more than 10 µM and 30 µM or less, the embryoid bodies could be effectively cultured into dorsal metencephalic tissue.

Accordingly, the embryoid bodies were selectively differentiated into dorsal metencephalic tissue as described above using the dorsal metencephalic tissue induction medium containing cyclopamine as the SHh signaling pathway inhibitor, at a concentration of approximately 20 µM, and the next step was prepared.

### [Basal surface-apex surface polarization] (embryoid bodies on days 7 to 10) (Day 7-10)

A basal surface-apex surface induction medium was prepared by adding approximately 2 vol% of Matrigel to the dorsal metencephalic tissue induction medium. Then, for about 3 days from day 4 after performing the selective differentiation into the dorsal metencephalic tissue, about 100 µl of the existing medium was removed about once a day, and about 100 µl of the basal surface-apex surface induction medium prepared as above was added, thereby inducing selective differentiation of the embryoid bodies into the dorsal metencephalic tissue and basal surface-apex surface polarization at the same time.

### [Cerebellar organoid maturation] (embryoid bodies on days 10 to 34) (Day 10-34)

5 ml of a cerebellar maturation medium containing a brain-derived neurotrophic factor (BDNF) and a glial cell-derived neurotrophic factor (GDNF) was added to each well of a well plate, and the basal surface-apex surface polarized embryoid bodies was transferred to each well. Then, about 4 ml of the existing medium was removed at intervals of about 2 days, and about 4 ml of a new cerebellar maturation medium was added, and the maturation process into the cerebellar organoid was performed (Day 10-34).

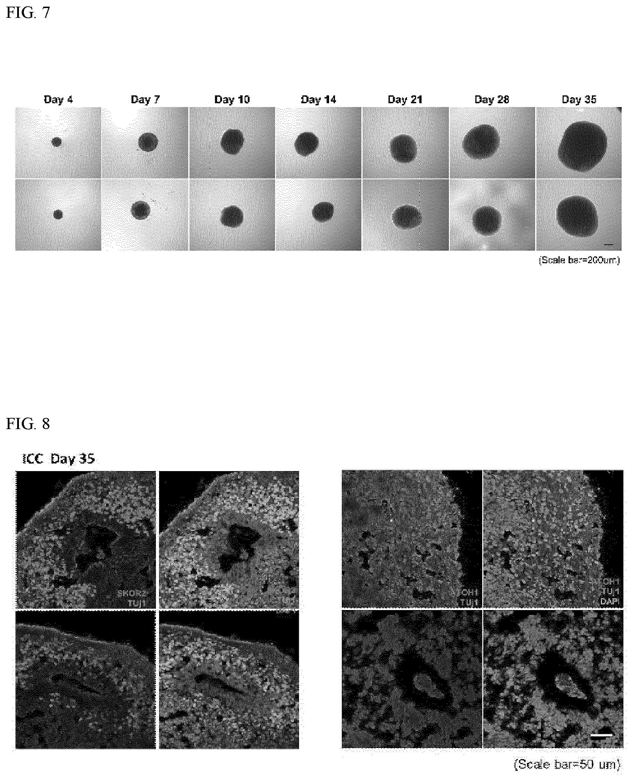

FIG. 6 shows a process of preparing a cerebellar organoid according to Example in a time series from embryoid bodies formation (Day 0). Furthermore, FIG. 7 shows a process of developing embryoid bodies in the process of preparing the cerebellar organoid according to Example observed through a phase microscope.

In addition, FIG. 8 shows results of observing a cerebellar organoid on Day 35 from the date of embryoid bodies formation according to the method for preparing the cerebellar organoid according to Example, using immunofluorescence staining. Specifically, according to the results of FIG. 8, it was confirmed that many cells expressing SKOR2, which marked progenitor cells of cerebellar-specific Purkinje neurons, and ATOH1, which marked progenitor cells of cerebellar-specific granule cells, were observed in the prepared cerebellar organoid.

## Claims

1. A method for preparing a cerebellar organoid comprising:
(A) a step of selective differentiation into hindbrain tissue, in which embryoid bodies formed from pluripotent stem cells are cultured in the presence of a hindbrain tissue induction medium containing an endogenous WNT secretion inhibitor and a WNT signaling agonist;
(B) a step of selective differentiation into dorsal metencephalic tissue, in which the embryoid bodies are cultured in the presence of a dorsal metencephalic tissue induction medium including a sonic hedgehog (SHh) signaling pathway inhibitor; and
(C) a basal surface-apex surface polarization step, in which polarization of the embryoid bodies are induced in the presence of a basal surface-apex surface induction medium including an extracellular matrix-based hydrogel.

2. The method of claim 1, wherein the step (A) of selective differentiation into the hindbrain tissue comprises
a1) a step of forming embryoid bodies from pluripotent stem cells in an embryoid body formation medium; and
a2) a step of adding a hindbrain tissue induction medium containing the endogenous WNT secretion inhibitor and the WNT signaling agonist to the embryoid body formation medium.

3. The method of claim 1, wherein the endogenous WNT secretion inhibitor includes at least one selected from the group consisting of IWP-2, LGK-974, ETC-159, GNF6231, WNT-C59, and WNT974.

4. The method of claim 1, wherein the hindbrain tissue induction medium includes an endogenous WNT secretion inhibitor in a concentration range that exhibits an effect equivalent to the effect exhibited by IWP-2 in a concentration range of 0.1 µM or more and 2 µM or less.

5. The method of claim 1, wherein the hindbrain tissue induction medium includes the WNT signaling agonist in a concentration range that exhibits an effect equivalent to the effect exhibited by CHIR99021 in a concentration range of more than 1 µM and less than 5 µM.

6. The method of claim 1, wherein in the step (A) of selective differentiation into the hindbrain tissue, at least one gene of LMX1A and LMX1B is expressed

7. The method of claim 1, wherein the dorsal metencephalic tissue induction medium includes the SHh signaling pathway inhibitor in a concentration range that exhibits an effect equivalent to the effect exhibited by cyclopamine in a concentration range of more than 10 µM and 30 µM or less.

8. The method of claim 1, wherein in the step (B) of selective differentiation into the dorsal metencephalic tissue, at least one gene of PAX7, OLIG3, ATOH1, BARHL1, PTF1A and SKOR2 is expressed.

9. The method of claim 1, wherein the basal surface-apex surface induction medium includes the extracellular matrix-based hydrogel in a concentration range of 1 vol% or more and 10 vol% or less.

10. The method of claim 1, wherein the step (A) of selective differentiation into the hindbrain tissue is performed for a period of 4 to 10 days,
the step (B) of selective differentiation into the dorsal metencephalic tissue is performed for a period of 4 to 10 days, and
the basal surface-apex surface polarization step (C) is performed for a period of 1 to 5 days.

11. The method of claim 10, wherein the step (A) of selective differentiation into the hindbrain tissue and the step (B) of selective differentiation into the dorsal metencephalic tissue are performed in an overlapping manner for a period of 1 to 5 days.

12. The method of claim 10, wherein the step (B) of selective differentiation into the dorsal metencephalic tissue and the basal surface-apex surface polarization step (C) are performed in an overlapping manner for a period of 1 to 5 days.

13. The method of claim 1, further comprising:
(D) a cerebellar organoid maturation step of maturing the embryoid body into the cerebellar organoid in the presence of a cerebellar maturation medium including at least one additive selected from the group consisting of a brain-derived neurotrophic factor (BDNF), a glial cell line-derived neurotrophic factor (GDNF), cyclic adenosine monophosphate (cAMP), and ascorbic acid.

14. The method of claim 1, wherein a ratio of cerebellar-compatible tissue in the entire prepared cerebellar organoid tissue is at least 90%.
